Europäisches Patentamt

European Patent Office

Office européen des brevets

(19)

(11) Veröffentlichungsnummer: **0 335 092**
**A2**

## (12) EUROPÄISCHE PATENTANMELDUNG

(21) Anmeldenummer: 89102562.9

(22) Anmeldetag: 15.02.89

(51) Int. Cl.⁴: $C07C\ 29/15$ , $B01J\ 23/34$

(30) Priorität: 26.03.88 DE 3810421

(43) Veröffentlichungstag der Anmeldung:
04.10.89 Patentblatt 89/40

(84) Benannte Vertragsstaaten:
AT BE CH DE ES FR GB GR IT LI LU NL SE

(71) Anmelder: **Union Rheinische Braunkohlen**
**Kraftstoff Aktiengesellschaft**
**Ludwigshafener Strasse o. Nr. Postfach 1663**
**D-5047 Wesseling(DE)**

(72) Erfinder: **Falter, Wolfgang**
**Todtleger 23**
**B-4731 Lichtenbusch(BE)**
Erfinder: **Keim, Wilhelm, Prof. Dr.**
**Brüsseler Ring 99**
**D-51oo Aachen(DE)**

(54) Verfahren zur katalytischen Herstellung eines Alkoholgemisches mit erhöhtem Isobutanolgehalt.

(57) Die vorliegende Erfindung betrifft ein Verfahren zur Herstellung eines Alkoholgemischs mit einem erhöhten Anteil an Isobutanol durch Umsetzung von CO und $H_2$ über Basen-modifizierten Zirkon/Zink/Mangankatalysatoren.

EP 0 335 092 A2

## Verbessertes Verfahren zur katalytischen Herstellung eines Alkoholgemischs mit erhöhtem Isobutanolgehalt

Die vorliegende Erfindung betrifft ein Verfahren zur Herstellung eines Alkoholgemischs mit einem erhöhten Anteil an Isobutanol durch Umsetzung von CO und $H_2$ über Basen-modifizierten Zirkon/Zink/Mangankatalysatoren.

Die Synthese Isobutanol enthaltender Alkoholgemische ist seit langem bekannt. Unter der Bezeichnung Isobutylölsynthese wurde ein Verfahren zur Gewinnung von Isobutanol aus Synthesegas von der BASF von 1935-52 großtechnisch durchgeführt. Zum Einsatz kamen hierbei Kalium-alkalisierte Hochdruck-ZnO-$Cr_2O_3$-Methanolkontakte (s. z.B. A. Palm in Chem. Technologie III. Organische Technologie I, K. Winnacker, L. Küchler, Carl Hauser Verlag, München, S. 359 ff. (1971) und dto. S. 572 ff. (1981). Die großtechnisch erzielten Ausbeuten von 11 - 14 % i-BuOH im Flüssigprodukt konnten durch Einsatz von $ZrO_2$ -Systemen, die mit Gallium-, Aluminium-, Chrom- und vor allem Indiumoxid mit Alkalimetallen versetzt waren, auf 20 - 40 % i-BuOH im Flüssigprodukt erhöht werden (s. DE-OS 928 045). Dieses Prinzip greift die Snamprogetti S.p.A. in ihren Patenten DE-OS 3 244 313, DE-OS 3 136 088, DE-OS 3 119 290 und FR 2 490 215 auf und erzielt nach einem speziellen Herstellungsverfahren mit Zn-$Cr_2O_3$-$K_2$O-Katalysatoren 23,2 % i-BuOH in der Flüssigphase.

Durch Alkali-modifizierte Niederdruck-Methanolkontakte CuO-ZnO-$K_2$O erzielt die Südchemie AG in ihrem Patent DE-OS 3 005 551 11,3 % Butanole. Die Standard Oil Co. beschreibt in EP-A-0 005 492, US 4 533 775 und US 4 559 316 Cu-Zr-Mn-Alkali-Katalysatoren mit Zusatz von seltenen Erden.

Das Institut Francais du Pétrole benutzt in den Offenlegungsschriften DE-OS 2 949 952, DE-OS 3 310 540, DE-OS 3 616 519, DE-OS 3 516 928, FR 2 543 945 und FR 2 504 916 CuO-ZnO-CoO- Alkali-Kontakte, die mit Pd-, seltenen Erden-, Na- und Li-Promotoren versetzt werden.

Das Octamix-Verfahren der Firma Lurgi verwendet alkalisierte Niederdruckmethanolkontakte und erzielt max. 6 - 7 % Butanole (s. Erdöl u. Kohle, Erdgas, Petrochemie; 38, 19-22 (1985) und DE-OS 3 403 492).

Die BP Co. erhält in EP 100 607 mit einem CuO-CoO-Alkali-Kontakt mit Ag, Ga, Zr, Zn, Th und Pr, Pt oder Ni als Promotor bei 19 %igem CO-Umsatz Ausbeuten an Butanolen von 7 %.

Kotowski erzielt mit CuO-ZnO-$Mn_2O_3$-Kontakten 10,9 % Butanole in der Flüssigphase (s. Chem. Stosow; 28 (1), 169-77 (1984)). Auch Klier erhält bei vergleichbar milden Reaktionsbedingungen (P = 7,5 MPa, T = 560-600 K) 15 % i-BuOH bei Selektivitäten von 6 % mittels CuO-ZnO-$Cs_2$O-Katalysatoren (s. Preprints Papers, Am.Chem.Soc., Div. Fuel Chem., 29 (5) 273-80 (1984).

Einen synthetischen Zeolithen und damit gänzlich andere Katalysatorsysteme beschreibt DD-OS 219 397 der VEB Leuna-Werke. Der Träger wird mit CuO, CoO, $Cr_2O_3$ oder FeO oder $Mn_xO_y$ beaufschlagt und enthält $K_2$O als basische Komponente. Es werden Selektivitäten von bis zu 6,3 % n-BuOH erzielt. Auch Goudeau berichtet in Comm.Eur.Communities, Eur (1985) Eur 10024, Energy Biomass, 959-62 (Fr.) über Alumosilikate des 13X Typs bzw. Mordenit-Typs, die mit Kupfer, Zink, Thorium und Cer imprägniert werden. Bei 523-673 K und 5 MPa erzielt er Anteile von 40 % i-BuOH in der Flüssigphase bei etwa 20 %igem CO-Umsatz. Leider werden flüssige Produkte wie Wasser aus der Bilanz herausgerechnet, so daß keine direkte Vergleichbarkeit mit anderen Katalysatorsystemen möglich ist.

In DE-OS 35 24 317 ist ein Verfahren zur Herstellung eines Alkoholgemisches mit erhöhtem Isobutanolgehalt beschrieben, in dem Katalysatoren verwendet werden, die $ZrO_2$ und/oder $Ce_2O_3$, Pd und eine Base enthalten. Zusätzlich können Elemente aus der Gruppe B, Al, Ga, In, Tl, Si, Ge, Sn, Pb, Cu, Zn, Se, Y, La, Ti, Hf, Th, V, Nb, Ta, Cr, Mo, Mn, Fe, Co, Ni, Ru, Rh, Os, Ir, Pt und seltene Erden mit Ausnahme von Cer enthalten sein.

In der gleichen Schrift wird ein weiterer Katalysator offenbart, der $ZrO_2$ und/oder $Ce_2O_3$ und Mangan sowie eine Base enthält.

In Tabelle 2 dieser Schrift sind Katalysatoren offenbart, die $ZrO_2$, $Ce_2O_3$, $K_2$O, MnO und Pd enthalten.

In Tabelle 5 sind Katalysatoren offenbart, die zusätzlich $In_2O_3$, $Fe_2$$O_3$, CuO, $Cr_2O_3$, $Al_2O_3$ und $La_2O_3$ enthalten.

Schließlich enthält Tabelle 7 Katalysatoren ohne MnO, welche $ZrO_2$, $K_2$O, Pd und die Metalloxide $ThO_2$, CuO, ZnO, MgO und $Cr_2O_3$ enthalten. Der höchste Isobutanolgehalt von 43,6 Gew.-% bei einer Raum/Zeit-Ausbeute (g/l.h) von 392 wird mit einem Katalysator aus $ZrO_2$, $K_2$O, MnO und Pd (K4, Tab.3) erhalten. Mit dem gleichen Katalysator wird unter geänderten Verfahrensbedingungen eine Raum/Zeit-Ausbeute von 1059 erhalten und ein Isobutanolgehalt von 30,2 Gew.-%.

Die Anmelderin hat nunmehr überraschend gefunden, daß noch bessere und zum Teil wesentlich bessere Ergebnisse erhalten werden, durch ein Verfahren zur Herstellung eines Alkoholgemischs mit erhöhtem Isobutanolgehalt aus CO und $H_2$ oder $CO_2$ und $H_2$ oder Mischungen von $CO_2$ und CO und $H_2$

oder anderen CO und/oder $CO_2$ und $H_2$ enthaltenden Gasen, dadurch gekennzeichnet, dar die Herstellung in Gegenwart eines Katalysators erfolgt, der folgende Komponenten enthält:

a, 5 - 80 Gew.-% Zirkon in elementarer Form und/oder in Form seiner Verbindungen,

b, 5 - 80 Gew.-% Zn in elementarer Form und/oder in Form seiner Verbindungen,

c, 1 - 80 Gew.-% Mn in elementarer Form und/oder in Form seiner Verbindungen,

d, 0,0001 - 10 Gew.-% einer basischen und/oder in eine basische Verbindung umwandelbaren Verbindung aus der Gruppe der Alkali- und/oder Erdalkalimetalle und/oder Ammoniak und/oder der basischen und/oder in eine basische Verbindung umwandelbaren Verbindungen des Ammoniaks.

Sehr gute Ergebnisse werden auch erhalten, wenn zusätzlich Pd in elementarer Form oder in Form seiner Verbindungen im Katalysator enthalten ist.

Obgleich im Stand der Technik Katalysatoren mit zahlreichen Kombinationen von Elementen bzw.deren Verbindungen, insbesondere deren Oxiden beschrieben sind, ist an keiner Stelle die erfindungsgemäße Kombination Zr-Zn-Mn erwähnt. Es war unvorhersehbar und für den Fachmann überraschend, daß gerade diese Kombination in Gegenwart von Basen herausragende Ergebnisse liefert.

Es ist bekannt, daß Alkoholgemische mit erhöhtem Isobutanolgehalt sehr gut als Oktanzahlverbesserer in Kraftstoffen geeignet sind; da sie im Gegensatz zu Methanol und Ethanol nicht zur Phasentrennung neigen, auch in Gegenwart von Feuchtigkeit bzw. kleineren Mengen Wasser.

Der im erfindungsgemäßen Verfahren eingesetzte Katalysator kann 5 - 80 Gew.-% Zirkon, bevorzugt 20 - 80 Gew.-% enthalten. Das Zirkon kann grundsätzlich in elementarer Form und/oder in Form seiner Verbindungen vorliegen, bevorzugt sind jedoch Katalysatoren, die Zirkonoxid $ZrO_2$ oder auch Zirkonate enthalten. Es kann sich auch um Gemische dieser genannten Komponenten handeln.

Zink kann ebenfalls im Katalysator in einer Menge von 5 - 80 Gew.-% und bevorzugt von 20 - 80 Gew.-% enthalten sein.

Auch das Zink kann grundsätzlich in elementarer Form und/oder in Form seiner Verbindungen enthalten sein. Bevorzugt ist ZnO, jedoch auch Zinkoxidhydrate oder Zinkate sind besonders geeignet. Es kann sich auch um Gemische dieser genannten Komponenten handeln.

Mangan kann mit 1 - 80 Gew.-% und bevorzugt mit 10 - 80 Gew.-% im Katalysator enthalten sein, sowohl in elementarer Form als auch in Form seiner Verbindungen. Bevorzugte Verbindungen sind insbesondere Manganoxide, Manganhydroxide, Manganoxidhydrate und Manganate.

Es kann sich auch um Gemische dieser genannten Komponenten handeln.

Als basische Komponenten im Katalysator sind die basischen Verbindungen der Alkalien und/oder Erdalkalien, insbesondere die Oxide und Hydroxide geeignet, aber auch andere basische Verbindungen, wie z.B. Carbonate, Hydrogencarbonate, Amide und dergl. sind erfindungsgemäß geeignet, jedoch auch Ammoniak und Amine und/oder basische Verbindungen des Ammoniaks und der Amine. Die basische Komponente, die auch aus Gemischen der genannten Verbindungen bestehen kann, ist in einer Menge von 0,0001 bis 10 Gew.-%, bevorzugt von 0,001 bis 5 Gew.-% und besonders bevorzugt von 0,05 - 5 Gew.-% im Katalysator enthalten.

Auch Verbindungen der Alkalien, Erdalkalien, des $NH_3$ und der Amine, welche basische Verbindungen bilden, können erfindungsgemäß eingesetzt werden, wie z.B. Metallhydride + $H_2O$ oder Ammoniumverbindungen, die durch Hydrolyse Ammoniumhydroxid bilden.

Im Falle des Zusatzes von Palladium enthält der Katalysator Palladium in einer Menge von 0,001 - 10 Gew.-%, bevorzugt von 0,01 - 2 Gew.-%.

Obgleich das Palladium im gebrauchsfertigen Katalysator im allgemeinen in elementarer Form vorliegt, kann es auch in Form von Verbindungen vorhanden sein, auch in Mischung von elementarer und gebundener Form.

Auch andere Elemente insbesondere der 1., 2., 3. und 8. Nebengruppe des Periodensystems können in gleicher Form und Menge als Einzelkomponente, im Gemisch mit Pd oder in anderen Gemischen untereinander im Katalysator enthalten sein. Beispiele sind Au, Ag, Cu, Sc, Y, Lanthaniden, Ru, Rh, Os, Ir und Pt. Entscheidend ist, daß die gemäß Anspruch 1 beanspruchte Kombination a-d im Katalysator enthalten ist.

Im erfindungsgemäßen Verfahren findet die Umsetzung mit CO und $H_2$ bzw. $CO_2$ und $H_2$ bzw. Mischungen von $CO_2$ und CO mit $H_2$ bzw. anderen CO und/oder $CO_2$ und $H_2$ enthaltenden Gasen in Gegenwart der erfindungsgemäßen Katalysatoren bei einer Temperatur von 420 -825 K, bevorzugt von 570 - 760 K und besonders bevorzugt von 680 - 730 K statt, bei einem Druck von 10 - 480 bar, bevorzugt von 20 - 400 bar und besonders bevorzugt bei 50 - 380 bar sowie einer GHSV (gas hourly space velocity) von 1.000 - 100.000 $h^{-1}$, bevorzugt von 2.000 - 75.000 $h^{-1}$ und besonders bevorzugt von 5.000 - 40.000 $h^{-1}$ statt.

Als Einsatzgas kann übliches Synthesegas verwendet werden, das überwiegend CO und $H_2$ enthält, jedoch können auch $CO_2$ und andere Gase wie beipielsweise $H_2O$-Dampf, $N_2$, $CH_4$ und andere enthalten sein. Bekanntlich erhöht sich aus stöchiometrischen Gründen bei relativ hohem CO-Anteil der Anteil an höheren Alkoholen im Produkt. Ein geeignetes Gasgemisch enthält beispielsweise CO : $H_2$ im Verhältnis 1:1. Das Verhältnis kann jedoch in weiten Grenzen etwa von 1:10 bis 10:1 variieren. Entsprechend der Stöchiometrie

$$4 \, CO + 8 \, H_2 \rightleftharpoons C_4H_9OH + 3 \, H_2O$$

bzw.: $7 \, CO + 5 \, H_2 \rightleftharpoons C_4H_9OH + 3 \, CO_2$

und der Koppelung der Nebenprodukte $CO_2$ und $H_2O$ über das Wassergasshiftgleichgewicht

$CO + H_2O \quad CO_2 + H_2$ läßt sich die Reaktion durch Wahl des $CO/H_2$-Verhältnisses wahlweise in die gewünschte Produktzusammensetzung leiten.

Bei hohen Wasserstoffanteilen erhält man bis zu 30 % Wasser im Reaktionsprodukt und hohe Anteile niederer Alkohole, insbesondere Methanol. Bei hohen CO-Anteilen im Einsatzgas steigt die Selektivität zu i-BuOH an. Es entsteht wenig Wasser im Reaktionsprodukt und viel konvertiertes $CO_2$. Bei $CO/H_2$-Verhältnissen >3/1 steigt der Anteil höherer Alkohole und der Anteil der Ketone im Reaktionsprodukt an. Die Gesamtraumzeitausbeuten sinken mit zunehmendem CO-Gehalt im Einsatzgas. Neben $CO/H_2$ hat sich der Zusatz von Alkoholen wie $C_1$-$C_6$-Alkoholen, insbesondere von Methanol, zu den Einsatzgasen als positiv für die Selektivität zu i-BuOH herausgestellt. Auch der Zusatz von n-Propanol und Methanol in reduzierender Atmosphäre erhöht die Ausbeute an i-BuOH. Neben Synthesegas kann das Methanol auch in anderen, bevorzugt reduzierenden Trägergasen dem Katalysator unter Reaktionsbedingungen zugeführt werden, aber auch ohne Verdünnung in Gasen kann Methanol direkt dem Katalysator zugeführt werden. Vorteilhaft bei Einsatz von Methanol im Einsatzgas ist die geringere positive Wärmetönung bei der Reaktion.

Die Darstellung der Katalysatoren erfolgt z.B. durch Copräcipitation oder sukzessive Präcipitation der entsprechenden Metallsalzlösungen, bevorzugt deren Nitrate. Die Fällung der Katalysatoren kann aber auch durch Komplexbildner, wie Hydroxy-, Poly-, Amino-Säuren oder Aminoalkoholen erfolgen.

Die Präcipitation der Katalysatoren erfolgt unter Temperatur-und pH-Kontrolle. Die Darstellung erfolgt bei 290 - 400 K, bevorzugt bei 330 - 373 K und pH-Werten von pH 3 - 13, bevorzugt pH 7 - 11. Dabei kann der pH-Wert durch Zugabe von Metallsalz- bzw. Basenlösung während der Fällung variiert werden, wird aber bevorzugt zur Erzeugung homogener Grundkörper konstant gehalten. Die Konzentration der Metallsalz- und Basenlösung kann in weiten Bereichen variiert werden, wird aber bevorzugt im Bereich von 0,1 - 5 $mol \, l^{-1}$ durchgeführt. Der Katalysator kann nach Präcipitation bei Temperaturen von 290 - 500 K, 0 - 50 Stunden, bevorzugt 5 - 25 Stunden altern. Der so erhaltene hydratisierte Grundkontakt kann gegebenenfalls gewaschen und nochmals gealtert werden. Nach der Formgebung in zylindrische Pellets wird der Kontakt bei Temperaturen von 330 - 430 K getrocknet und thermisch bei Temperaturen von 420 - 875 K, bevorzugt 500 - 700 K in Luft oder Inertgas über einen Zeitraum von 1 - 10 Stunden aktiviert und kann anschliessend mit Wasserstoff bei erhöhtem Druck (2 - 250 bar) und erhöhter Temperatur (400 - 700 K) reduziert werden. Die Formgebung kann mittels Extrudern oder mittels Pressen oder anderer Geräte erfolgen. Während der Trocknung, Kalzinierung und Reduktion wird das Gewicht des hydratisierten Niederschlags im allgemeinen auf etwa 10 - 15 % der ursprünglichen Katalysatormenge reduziert (TGA). Die Imprägnierung mit Komponenten aus den Nebengruppen des Periodensystems erfolgt vor oder nach der Kalzinierung. Die Edelmetalle werden im allgemeinen als lösliche Verbindungen (z.B. Nitrate) in Wasser oder als organische Verbindungen (z.B. Acetylacetonate oder Komplexe wie Carbonylkomplexe) in organischen Lösungsmitteln (z.B. Alkohole oder Acetonitril) angereichert. Dabei können beispielsweise die incipient wetness Methode, eine langsame Imprägnierung oder andere geeignete Verfahren angewendet werden. Die Edelmetalle können auch durch Copräcipitation oder sukzessive Fällung in den Kontakt eingebaut werden, die Imprägnierung der Oberfläche ist jedoch bevorzugt. Die organischen oder anorganischen Reste des Edelmetalls können durch Kalzinierung thermisch zerstört werden. Werden Träger verwendet, so kann nach Aufbringen der gewünschten Komponenten durch Tränken ebenfalls eine Aktivierung durch Kalzinieren durchgeführt werden. Ferner kann dem Fällungsgemisch ein Bindemittel (wie z.B. Graphit, Stearinsäure und deren Ester, Cellulose oder andere pflanzliche Materialien) zugegeben werden, wobei durch gezielte Zersetzung oder durch Abtrennen eine bestimmte Porengröße erzeugt werden kann. Die mit Edelmetallen beschichteten Katalysatoren werden wahlweise direkt in die Reaktion eingesetzt, bevorzugt aber nochmals getrocknet, kalziniert und reduziert. Die Reduktion geschieht in reduzierenden Gasen, bevorzugt Wasserstoff mit Vol.-Anteilen von 0,1 - 100 %, bevorzugt 50 - 100 % bei Drücken von 2 - 250 bar, bevorzugt 10 - 100 bar mit Volumengeschwindigkeiten von 500 - 100.000 $h^{-1}$, bevorzugt 5.000 - 20.000 $h^{-1}$. Die beschriebenen Herstellungsmethoden sind beispielhaft, jedoch nicht limitierend. Auch andere Herstellungsmethoden sind für die Herstellung der erfindungsgemäßen Katalysatoren geeignet.

Mit Hilfe der folgenden, in Tabellen zusammengefaßten Versuche wird die vorliegende Erfindung näher erläutert. Sofern nicht anders angegeben, wurde mit einem $CO/H_2$-Verhältnis von 1:1 gearbeitet.

In den Tabellen sind die Angaben mit denen des Standes der Technik gemäß DE-OS 35 24 317 vergleichbar. Diese Anmeldung wurde von dem gleichen Anmelder eingereicht wie die vorliegende Erfindung.

In Tabelle 1 ist das Verhältnis $ZrO_2$:$ZnO$:$Mn_xO_y$ variiert. Druck, Temperatur und Fällungs-pH sind gleich. Als Basen werden KOH, LiOH und $NH_3$ verwendet.

Die Versuche zeigen, daß ein Verhältnis von 1:1:1 (bezogen auf Molmengen der eingesetzten Nitrate) sowohl zu einer guten Raumzeitausbeute führt, als auch zu einer vergleichsweise hohen Isobutanolselektivität, die mit 44 Gew.-% höher liegt als der beste Versuch der DE-OS 35 24 317 aus Tabelle 3 mit 43,6 Gew.-% und einer Raumzeitausbeute von 392 g/l.h.

Tabelle 1

| Abhängigkeit der Raumzeitausbeute und Isobutanol-Selektivität vom Mengenverhältnis $ZrO_2$:$ZnO$: $Mn_xO_y$ mit $K_2O$, $Li_2O$ und $NH_3$ als Base | | | | | | | |
|---|---|---|---|---|---|---|---|
| Katalysatorkennzeichen | WF 77 | WF 85 | WF 79 | WF 93 | WF 162 | WF 163 | WF 164 |
| Mengenverhältnis* $ZrO_2$-$ZnO$-$Mn_xO_y$ | 1:1:1 | 2:2:1 | 2:1:2 | 2:2:1 | 1:4:3 | 1:6:1 | 5:1:1 |
| Reaktionstemperatur, K | 700 | 700 | 700 | 688 | 700 | 700 | 700 |
| Reaktionsdruck, bar | 250 | 250 | 250 | 250 | 250 | 250 | 250 |
| GHSV/$h^{-1}$ | 20.000 | 20.000 | 20.000 | 20.000 | 20.000 | 20.000 | 20.000 |
| Raumzeitausbeute g/l.h (Alkohole) | 920 | 810 | 715 | 280 | 320 | 370 | 1005 |
| Isobutanol-Selektivität, Gew.-% | 44 | 40 | 39 | 43 | 27 | 28 | 41 |
| Fällungs-pH | 9 | 9 | 9 | 9 | 9 | 9 | 9 |
| Base | $K_2O$ | $K_2O$ | $Li_2O$ | $NH_3$ | $K_2O$ | $K_2O$ | $K_2O$ |

* bezogen auf eingesetzte Molmengen an Metallverbindungen

Tabelle 2

| Tabelle 2 zeigt den Einfluß der Reaktionstemperatur. Als Katalysator wurde $ZrO_2$-$ZnO$-$Mn_xO_y$-$K_2O$ im Verhältnis 1:1:1 eingesetzt. Der Fällungs-pH betrug 1 . Die Tabelle zeigt, daß die Raumzeitausbeute bei ca. 675 K ein Maximum von 850 g/l.h durchläuft, die Isobutanolselektivität jedoch bei 700-715 K auf 55 Gew.-% ansteigt und bei noch höherer Temperatur wieder abfällt. | | | | | | |
|---|---|---|---|---|---|---|
| Einfluß der Reaktionstemperatur auf Raumzeitausbeute und Isobutanolselektivität | | | | | | |
| Katalysatorkennzeichen | | | WF 115 | | | |
| Mengenverhältnis* $ZrO_2$-$ZnO$-$Mn_xO_y$ | | | 1:1:1 | | | |
| Reaktionstemperatur K | 600 | 625 | 650 | 675 | 700 | 715 | 725 |
| Reaktionsdruck, bar | | | 250 | | | |
| GHSV/$h^{-1}$ | | | 20.000 | | | |
| Raumzeitausbeute g/l.h (Alkohole) | 550 | 685 | '725 | 850 | 855 | 710 | 625 |
| Isobutanol-Selektivität, Gew.-% | 3 | 18 | 29 | 45 | 55 | 55 | 52 |
| Fällungs-pH | | | 11 | | | |
| Base | | | $K_2O$ | | | |

* bezogen auf eingesetzte Molmengen an Metallverbindungen

Tabelle 3

Mit dem gleichen Katalysator wurde gemäß Tabelle 3 der Einfluß des Drucks untersucht. Während die Raumzeitausbeute bis ca. 300 bar auf 805 g/l.h ansteigt, fällt die Isobutanolselektivität von 58 Gew.-% auf 38 Gew.-% bei 350 bar ab.

Einfluß des Reaktionsdrucks auf Raumzeitausbeute und Isobutanolselektivität

| | | | | | | |
|---|---|---|---|---|---|---|
| Katalysatorkennzeichen | | | | WF 115 | | |
| Mengenverhältnis* $ZrO_2$-ZnO-$Mn_xO_y$ | | | | 1:1:1 | | |
| Reaktionstemperatur K | | | | 700 | | |
| Reaktionsdruck, bar | 50 | 100 | 150 | 250 | 300 | 350 |
| GHSV/$h^{-1}$ | | | | 20.000 | | |
| Raumzeitausbeute g/l.h (Alkohole) | 240 | 445 | 565 | 755 | 805 | 765 |
| Isobutanol-Selektivität, Gew.-% | 58 | 58 | 55 | 55 | 48 | 38 |
| Fällungs-pH | | | | 11 | | |
| Base | | | | $K_2O$ | | |

* bezogen auf eingesetzte Molmengen an Metallverbindungen

Tabelle 4

Tabelle 4 betrifft den Einfluß der Gasbelastung bei Einsatz des gleichen Katalysators. Das Maximum der Raumzeitausbeute liegt bei einer GHSV/$h^{-1}$ von 20.000-25.000, während das Maximum der Isobutanolselektivität von 61 Gew.-% bei einer GHSV/$h^{-1}$ von ca. 10.000 liegt.

Einfluß der Gasbelastung auf Raumzeitausbeute und Isobutanolselektivität

| | | | | | | |
|---|---|---|---|---|---|---|
| Katalysatorkennzeichen | | | WF 115 | | | |
| Mengenverhältnis* $ZrO_2$-ZnO-$Mn_xO_y$ | | | 1:1:1 | | | |
| Reaktionstemperatur K | | | 700 | | | |
| Reaktionsdruck, bar | | | 250 | | | |
| GHSV/$h^{-1}$ | 5.000 | 10.000 | 15.000 | 20.000 | 25.000 | 30.000 |
| Raumzeitausbeute g/l.h (Alkohole) | 545 | 680 | 710 | 755 | 760 | 660 |
| Isobutanol-Selektivität, Gew.-% | 59 | 61 | 56 | 55 | 50 | 36 |
| Fällungs-pH | | | 11 | | | |
| Base | | | $K_2O$ | | | |

* bezogen auf eingesetzte Molmengen an Metallverbindungen

Tabelle 5

In Tabelle 5 ist der Einfluß der verwendeten Base dargestellt. Es wird deutlich, daß sich bei gleichem Fällungs-pH von 9, $K_2O$ und $Cs_2O$ günstig auf die Raumzeitausbeuten auswirken, während $Na_2O$ bei en angegebenen Bedingungen zu der höchsten Isobutanolselektivität von 62 Gew.-% führt. Demgemäß sollten mit gemischten Basen noch bessere Ergebnisse erhältlich sein.

Abhängigkeit von Raumzeitausbeute und Isobutanolselektivität von der verwendeten Base

| Katalysatorkennzeichen | WF 77 | WF 89 | WF 95 | WF 97 | WF 101 | WF 103 |
|---|---|---|---|---|---|---|
| Mengenverhältnis* $ZrO_2$-$ZnO$-$Mn_xO_y$ | 1:1:1 | 1:1:1 | 1:1:1 | 1:1:1 | 1:1:1 | 1:1:1 |
| Reaktionstemperatur, K | 700 | 700 | 700 | 700 | 700 | 700 |
| Reaktionsdruck, bar | 250 | 250 | 250 | 250 | 250 | 250 |
| GHSV/h$^{-1}$ | 20.000 | 20.000 | 20.000 | 20.000 | 20.000 | 20.000 |
| Raumzeitausbeute g/l.h (Alkohole) | 810 | 680 | 850 | 510 | 355 | 465 |
| Isobutanol-Selektivität, Gew.-% | 44 | 62 | 50 | 40 | 35 | 60 |
| Fällungs-pH | 9 | 9 | 9 | 9 | 9 | 9 |
| Base | $K_2O$ | $Na_2O$ | $Cs_2O$ | MgO | SrO | BaO |

* bezogen auf eingesetzte Molmengen an Metallverbindungen

In den Tabellen 6 und 7 ist der Einfluß des Fällungs-pH's bei Verwendung von $K_2O$ und $Li_2O$ als Basen wiedergegeben. Es zeigt sich, daß sich im Falle von $K_2O$, ein Fällungs-pH von 7 oder 11 besonders günstig auf Raumzeitausbeute und Isobutanolselektivität, mit über 50 Gew.-% i-Butanol in der Flüssigphase auswirkt, während mit $Li_2O$ als Base das Maximum der Raumzeitausbeute bei ca. 835 g/l.h und einem pH 8 liegt, während in Übereinstimmung mit $K_2O$ als Base, die Isobutanolselektivität mit steigendem Fällungs-pH bis auf 64 Gew.-% zunimmt.

<u>Tabelle 6</u>

Einfluß des Fällungs-pH's auf Raumzeitausbeute und Isobutanolselektivität mit $K_2O$ als Base

| Katalysator-kennzeichen | WF 105 | WF 107 | WF 109 | WF 111 | WF 77 | WF 113 | WF 115 | WF 117 |
|---|---|---|---|---|---|---|---|---|
| Mengenverhältnis* $ZrO_2$-ZnO-$Mn_xO_y$ | 1:1:1 | 1:1:1 | 1:1:1 | 1:1:1 | 1:1:1 | 1:1:1 | 1:1:1 | 1:1:1 |
| Reaktionstemperatur K | 700 | 700 | 700 | 700 | 700 | 700 | 700 | 700 |
| Reaktionsdruck, bar | 250 | 250 | 250 | 250 | 250 | 250 | 250 | 250 |
| GHSV/h$^{-1}$ | 20.000 | 20.000 | 20.000 | 20.000 | 20.000 | 20.000 | 20.000 | 20.000 |
| Raumzeitausbeute g/l.h (Alkohole) | 165 | 240 | 380 | 895 | 920 | 1045 | 905 | 240 |
| Isobutanol-Selektivität, Gew.-% | 21 | 39 | 58 | 42 | 44 | 46 | 51 | 27 |
| Fällungs-pH | 3 | 5 | 7 | 8 | 9 | 10 | 11 | 13 |
| Base | $K_2O$ | $K_2O$ | $K_2O$ | $K_2O$ | $K_2O$ | $K_2O$ | $K_2O$ | $K_2O$ |

* bezogen auf eingesetzte Molmengen an Metallverbindungen

EP 0 335 092 A2

Tabelle 7

| Einfluß des Fällungs-pH's auf Raumzeitausbeute und Isobutanolselektivität mit $Li_2O$ als Base | | | | |
|---|---|---|---|---|
| Katalysatorkennzeichen | WF 142 | WF 144 | WF 146 | WF 148 |
| Mengenverhältnis* $ZrO_2$-ZnO-$Mn_xO_y$ | 1:1:1 | 1:1:1 | 1:1:1 | 1:1:1 |
| Reaktionstemperatur K | 715 | 715 | 715 | 715 |
| Reaktionsdruck, bar | 250 | 250 | 250 | 250 |
| GHSV/h$^{-1}$ | 20.000 | 20.000 | 20.000 | 20.000 |
| Raumzeitausbeute g/l.h (Alkohole) | 550 | 835 | 755 | 440 |
| Isobutanol-Selektivität, Gew.-% | 18 | 49 | 53 | 64 |
| Fällungs-pH | 6 | 8 | 10 | 11 |
| Base | $Li_2O$ | $Li_2O$ | $Li_2O$ | $Li_2O$ |

* bezogen auf eingesetzte Molmengen an Metallverbindungen

Tabelle 8

| Bei den in Tabelle 8 zusammengestellten Versuchen wurde ein Katalystor eingesetzt aus $ZrO_2$-ZnO-MnO im Verhältnis 1:1:1. Der Fällungs-pH mit $K_2O$ als Base lag bei 9. Zusätzlich wurden dem Katalysator Elemente aus der 1., 2. und 8. Nebengruppe zugesetzt. Weitaus am günstigsten erwies sich Pd, verglichen mit Au, Pt, Ru und Cd. $ZrO_2$-ZnO-$Mn_xO_y$ 1:1:1* | | | | | |
|---|---|---|---|---|---|
| Einfluß eines zusätzlichen Elements aus der 1., 2. und 8. Nebengruppe auf die Raumzeitausbeute und Isobutanolselektivität Fällungs-pH 9, Base: $K_2O$ | | | | | |
| Katalysatorkennzeichen | WF 104 | WF 119 | WF 122 | WF 114 | WF 67 |
| Zusatzelement (0,25 Gew.-%) | Pd | Au | Pt | Ru | Cu** |
| Reaktionstemperatur K | 700 | 700 | 700 | 700 | 700 |
| Reaktionsdruck, bar | 250 | 250 | 250 | 250 | 850 |
| GHSV/h$^{-1}$ | 20.000 | 20.000 | 20.000 | 20.000 | 20.000 |
| Raumzeit-Ausbeute g/l.h | 810 | 595 | 245 | 225 | 610 |
| Isobutanol-Selektivität, Gew.-% | 54,0 | 47,6 | 34,0 | 22,2 | 44,3 |

* 1:1:1 bezogen auf Molmengen eingesetzter Metallverbindungen

Tabelle 9

| In den folgenden Versuchen wurde die Kombination $ZrO_2$-ZnO-$Mn_xO_y$-Pd näher untersucht. |
|---|
| In Tabelle 9 ist die Gasbelastung und ihre Auswirkung auf Raumzeitausbeute und Isobutanolselektivität wiedergegeben. Es wurde ein Katalysator mit 0,25 Gew.-% Pd und einem $ZrO_2$/ZnO/$Mn_xO$ b>y-Verhältnis von 1:1:1* verwendet. Als Base diente $K_2O$. |
| Die Tabelle zeigt, daß das Maximum der Raumzeitausbeute bei einer GHSV/$h^{-1}$ von 20.000 bis 25.000 liegt, nämlich von 1010 bis 1095 g/l.h. Die Isobutanolselektivität ist bei einer GHSV/$h^{-1}$ von 5.000 bis 15.000 ko stant bei ca. 62 Gew.-% und fällt dann ab auf 59,4 Gew.-% bei einer GHSV/$h^{-1}$ von 25.000. |

Einfluß der Gasbelastung auf die Raumzeitausbeute und die Isobutanolselektivität mit dem Katalysator: $ZrO_2$-ZnO-$Mn_xO_y$ 1:1:1, 0,25 Gew.-% Pd, Base: $K_2O$

| Katalysatorkennzeichen | WF 116 | WF 116 | WF 116 | WF 116 | WF 116 | WF 116 |
|---|---|---|---|---|---|---|
| Temperatur K | 715 | 715 | 715 | 715 | 715 | 715 |
| Druck bar | 250 | 250 | 250 | 250 | 250 | 250 |
| GHSV/$h^{-1}$ | 5.000 | 10.000 | 15.000 | 20.000 | 25.000 | 30.000 |
| Fällungs-pH | 10 | 10 | 10 | 10 | 10 | 10 |
| Raumzeit-Ausbeute g/l.h | 685 | 935 | 990 | 1010 | 1095 | 915 |
| Isobutanol-Selektivität, Gew.-% | 62,5 | 62,0 | 62,7 | 61,1 | 59,4 | 29,1 |

* bezogen auf Molmengen eingesetzter Metallverbindungen

In Tabelle 10 ist der Einfluß des Fällungs-pH's bei gleicher Katalysatorzusammensetzung wiedergegeben.

Hier ist der Bereich von pH 9-10 besonders günstig bezüglich Raumzeitausbeute und Isobutanolselektivität.

Tabelle 10

Einfluß des Fällungs-pH's auf Raumzeitausbeute und Isobutanol-Selektivität mit dem Katalysator: $ZrO_2$-ZnO-$Mn_xO_y$  1:1:1, bezogen auf Molmengen eingesetzter Metallverbindungen, 0,25 Gew.-% Pd, Base: $K_2O$

| Katalysator-kennzeichen | WF 106 | WF 108 | WF 110 | WF 112 | WF 114 | WF 116 | WF 118 | WF 156 |
|---|---|---|---|---|---|---|---|---|
| Temperatur K | 700 | 700 | 700 | 700 | 700 | 700 | 700 | 700 |
| Druck bar | 250 | 250 | 250 | 250 | 250 | 250 | 250 | 250 |
| GHSV/h$^{-1}$ | 20.000 | 20.000 | 20.000 | 20.000 | 20.000 | 20.000 | 20.000 | 20.000 |
| Fällungs-pH | 3 | 5 | 7 | 8 | 9 | 10 | 13 | ohne Base |
| Raumzeit-Ausbeute g/l.h | 160 | 379 | 515 | 830 | 810 | 950 | 250 | 2 |
| Isobutanol-Selekt. Gew.-% | 20,7 | 48,3 | 63,4 | 37,3 | 54,0 | 51,5 | 12,5 | 0 |

EP 0 335 092 A2

Tabelle 11

| Schließlich gibt Tabelle 11 den Einfluß der Pd-Menge im Katalysator wieder. Günstige Pd-Mengen liegen bei 0,15-0,25 Gew.-%. | | | | | |
|---|---|---|---|---|---|
| Einfluß der zugesetzten Pd-Menge auf die Raumzeitausbeute und die Isobutanolselektivität mit dem Katalysator $ZrO_2$-ZnO-m$N_xO_y$ 1:1:1*, Base: $Li_2O$ | | | | | |
| Katalysatorkennzeichen | WF 150 | WF 151 | WF 149 | WF 152 | WF 153 |
| Temperatur K | 700 | 700 | 700 | 700 | 700 |
| Druck bar | 250 | 250 | 250 | 250 | 250 |
| GHSV/h$^{-1}$ | 20.000 | 20.000 | 20.000 | 20.000 | 20.000 |
| zugesetzte Menge an Pd in % | 0,05 | 0,15 | 0,25 | 0,5 | 1,0 |
| Fällungs-pH | 11 | 11 | 11 | 11 | 11 |
| Raumzeit-Ausbeute g/l.h | 500 | 700 | 735 | 510 | 530 |
| Isobutanol-Selektivität, Gew.-% | 47,0 | 46,9 | 41,3 | 17,9 | 18,6 |

* bezogen auf eingesetzte Molmengen an Metallverbindungen

Die Variation des CO/$H_2$-Verhältnisses gibt Tabelle 12 wieder.

Es wurde ein $ZrO_2$-ZnO-$Mn_xO_y$-Pd-Katalysator eingesetzt (0,25 Gew.-% Pd, Fällungs-pH 9).

Das günstigste Ergebnis bezüglich Raumzeitausbeute und Isobutanolselektivität wurde mit einem CO/$H_2$-Verhältnis von 1:2 erzielt.

Mit zunehmendem CO/$H_2$-Verhältnis geht die Raumzeitausbeute stark zurück, während die Isobutanolselektivität etwas ansteigt.

Tabelle 12

| CO/$H_2$ | 1:2 | 1:1 | 2:1 | 3:1 |
|---|---|---|---|---|
| Temperatur K | 700 | 700 | 700 | 700 |
| Druck, bar | 25 | 25 | 25 | 25 |
| GHSV/h$^{-1}$ | 20.000 | 20.000 | 20.000 | 20.000 |
| Raumzeit-Ausbeute g/l.h | 635 | 475 | 450 | 240 |
| Isobutanol-Selektivität, Gew.-% | 50,8 | 47,7 | 54,2 | 54,2 |

Tabelle 13

| Tabelle 13 zeigt, daß sich durch Zusatz von Methanoldampf Raumzeitausbeute und Selektivität zu Isobutylalkohol stark beeinflussen lassen, (gleicher Katalysator). | | | | |
|---|---|---|---|---|
| CO/$H_2$(1:1):MeOH | 1:0 | 3:1 | 2:1 | 1:1 |
| Temperatur K | 700 | 700 | 700 | 700 |
| Druck, bar | 10 | 10 | 10 | 10 |
| GHSV/h$^{-1}$ | 20000 | 15000 | 13500 | 10000 |
| Raumzeitausbeute g/l.h | 295 | 700 | 1690 | 3960 |
| Isobut.-Selek,Gew.-% | 30,9 | 38,1 | 45,2 | 57,2 |

Gute Ergebnisse können auch erzielt werden, wenn sich die erfindungsgemäßen Katalysatoren auf Trägern insbesondere neutralen bzw. basischen Metalloxiden befinden.

Ferner werden ebenfalls gute Ergebnisse erhalten mit $CO_2$/$H_2$ und CO/$CO_2$/$H_2$-Gasgemischen.

Die erfindungsgemäßen Ergebnisse zeigen, daß sich mit dem beanspruchten Katalysatorsystem die Selektivitäten bezüglich Isobutanol ganz erheblich steigern lassen bei befriedigenden bis sehr guten Raumzeitausbeuten.

**Ansprüche**

1. Verfahren zur katalytischen Herstellung eines Alkoholgemischs mit erhöhtem Isobutanolgehalt aus CO und $H_2$ oder $CO_2$ und $H_2$ oder Mischungen von $CO_2$ und CO und $H_2$ oder anderen CO und/oder $CO_2$ und $H_2$ enthaltenden Gasen, dadurch gekennzeichnet, daß die Herstellung des Alkoholgemischs in Gegenwart eines Katalysators erfolgt, der folgende Komponenten enthält:
a, 5 - 80 Gew.-% Zirkon in elementarer Form und/oder in Form seiner Verbindungen,
b, 5 - 80 Gew.-% Zn in elementarer Form und/oder in Form seiner Verbindungen,
c, 1 - 80 Gew.-% Mn in elementarer Form und/oder in Form seiner Verbindungen,
d, 0,0001 - 10 Gew.-% wenigstens einer basischen und/oder in eine basische Verbindung umwandelbaren Verbindung aus der Gruppe der Alkali- und/oder Erdalkalimetalle und/oder Ammoniak und/oder der basischen und/oder in eine basische Verbindung umwandelbaren Verbindungen des Ammoniaks.

2. Verfahren nach Anspruch 1, dadurch gekennzeichnet, daß in Gegenwart eines Katalysators gearbeitet wird, der bevorzugt 20 - 80 Gew.-% Zirkon enthält.

3. Verfahren nach den Ansprüchen 1 und 2, dadurch gekennzeichnet, daß in Gegenwart eines Katalysators gearbeitet wird, der bevorzugt 20 - 80 Gew.-% Zn enthält.

4. Verfahren nach den Ansprüchen 1 - 3, dadurch gekennzeichnet, daß in Gegenwart eines Katalysators gearbeitet wird, der bevorzugt 10 - 80 Gew.-% Mangan enthält.

5. Verfahren nach den Ansprüchen 1 - 4, dadurch gekennzeichnet, daß in Gegenwart eines Katalysators gearbeitet wird, der bevorzugt 0,001 - 5 Gew.-% der Komponente d, enthält.

6. Verfahren nach den Ansprüchen 1 - 5 , dadurch gekennzeichnet, daß in Gegenwart eines Katalysators gearbeitet wird, der zusätzlich wenigstens ein Element aus den Gruppen 1., 2., 3. und 8, Nebengruppe des Periodensystems in einer Menge von 0,001 bis 10 Gew.-%, bevorzugt von 0,01 - 2 Gew.-% in elementarer Form und/oder in Form seiner Verbindungen enthält.

7. Verfahren nach den Ansprüchen 1 - 5, dadurch gekennzeichnet, daß in Gegenwart eines Katalysators gearbeitet wird, der zusätzlich 0,001 bis 10. Gew.-%, bevorzugt 0,01 - 2 Gew.-% Palladium in elementarer Form und/oder in Form seiner Verbindungen enthält.

8. Verfahren nach den Ansprüchen 1 - 7, dadurch gekennzeichnet, daß in Gegenwart eines Katalysators gearbeitet wird, der die Komponenten Zirkon, Zn und Mangan in Form von Oxiden enthält.

9. Verfahren nach den Ansprüchen 1 - 8, dadurch gekennzeichnet, daß bei einer Temperatur von 420 - 825 K, bevorzugt von 570 - 760 K und besonders bevorzugt von 680 - 730 K, bei einem Druck von 10 - 480 bar, bevorzugt von 20 bis 400 bar und besonders bevorzugt von 50 - 380 bar und einer GHSV (gas hourly space velocity) von 1.000 - 100.000 $h^{-1}$ bevorzugt von 2.000 - 75.000 $h^{-1}$ und besonders bevorzugt von 5.000 - 40.000 $h^{-1}$ gearbeitet wird.

10. Verfahren nach den Ansprüchen 1 - 9, dadurch gekennzeichnet, daß ein $CO/H_2$-Gemisch im Verhältnis 3:1 bis 1:3 eingesetzt wird.

11. Verfahren nach den Ansprüchen 1 - 10, dadurch gekennzeichnet, daß zu dem Einsatzprodukt Alkohole zugesetzt werden.

12. Verfahren nach den Ansprüchen 1 - 11, dadurch gekennzeichnet, daß zu den Einsatzprodukt Methanol zugesetzt wird.

13. Katalysator, dadurch gekennzeichned, daß derselbe folgende Komponenten enthält:
a) 5 - 80 Gew.-% Zirkon in elementarer Form und/oder in Form seiner Verbindungen,
b) 5 - 80 Gew.-% Zn in elementarer Form und/oder in Form seiner Verbindungen,
c) 1 - 80 Gew.-% Mn in elementarer Form und/oder in Form seiner Verbindungen,
d) 0,0001 - 10 Gew.-% wenigstens einer basischen und/oder in eine basische Verbindung umwandelbaren Verbindung aus der Gruppe der Alkali- und/oder Erdalkalimetalle und/oder Ammoniak und/oder der basischen und/oder in eine basische Verbindung umwandelbaren Verbindungen des Ammoniaks.

14. Katalysator nach Anspruch 13, dadurch gekennzeichnet, daß derselbe 20 - 80 Gew.-% Zr enthält.

15. Katalysator nach den Ansprüchen 13 und 14, dadurch gekennzeichnet, daß derselbe 20 - 80 Gew.-% Zn enthält.

16. Katalysator nach den Ansprüchen 13 - 15, dadurch gekennzeichnet, daß derselbe 1 0 - 80 Gew.-% Mn enthält.

17. Katalysator nach den Ansprüchen 13 - 16, dadurch gekennzeichnet, daß derselbe 0, 001 - 5 Gew. -% der Komponente d enthält.

18. Katalysator nach den Ansprüchen 13 - 17, dadurch gekennzeichnet, daß derselbe zusätzlich wenigstens ein Element aus den Gruppen 1., 2., 3. und 8, Nebengruppe des Periodensystems in einer Menge von 0, 001 bis 10 Gew.-%, bevorzugt von 0,01 - 2 Gew.-% in elementarer Form und/oder in Form seiner Verbindungen enthält.

19. Katalysator nach den Ansprüchen 13 - 17, dadurch gekennzeichnet, daß derselbe zusätzlich 0, 001 bis 10 Gew.-%, bevorzugt 0,01 - 2 Gew.-% Palladium in elementarer Form und/oder in Form seiner Verbindungen enthält.

20. Katalysator nach den Ansprüchen 13 - 19, dadurch gekennzeichnet, daß derselbe die Komponenten Zirkon, Zink und Mangan in Form von Oxiden enthält.